# EUROPEAN PATENT SPECIFICATION

(11) **EP 3 756 659 B1**
(45) Date of publication and mention of the grant of the patent: **06.12.2023**
(21) Application number: 20186427.9
(22) Date of filing: 29.08.2016
(51) Int. Cl.: A61K 9/70, A61K 47/02, A61K 47/12, A61K 47/18, A61K 31/433, A61P 25/04

(54) **PATCH PREPARATION CONTAINING AN ACID SCAVENGER**
PFLASTERPRÄPARAT MIT EINEM SÄUREFÄNGER
PRÉPARATION PHARMACEUTIQUE COLLANTE COMPRENANT UN CAPTEUR D'ACIDE

(30) Priority: 29.08.2015 WO PCT/JP2015/074552
(43) Date of publication of application: 30.12.2020
(62) Divisional of application: 16841800.2
(73) Proprietor: MEDRx Co., Ltd., Higashikagawa-shi Kagawa 769-2712 (JP)
(72) Inventor: Hamamoto, Hidetoshi, Higashikagawa-shi, Kagawa 769-2712 (JP); Tanimoto, Takahiro, Higashikagawa-shi, Kagawa 769-2712 (JP)
(74) Representative: Vossius & Partner Patentanwälte Rechtsanwälte mbB

(56) References cited:
- EP-A1- 0 295 411
- EP-A1- 1 591 110
- EP-A1- 2 255 809
- EP-A1- 2 716 287
- EP-A1- 2 865 376
- EP-A2- 0 315 219
- WO-A2-2010/083035

## Description

### Technical Field

The present invention relates to a patch preparation containing an acid addition salt of a basic medicament, i.e. tizanidine, as active ingredient. More specifically, the present invention relates to a patch preparation in which a scavenger of acid attached to the medicament is contained to encourage the production of free base.

### Background Art

As an example of techniques for accelerating the transdermal absorption of a medicament, the technique for dissolving the medicament in a fatty acid-based ionic liquid has been known (Patent Document 1).

Basic medicament is often distributed in the form of an acid addition salt such as hydrochloride salt in view of the stability and handling. However, it has been known that such acid addition salt has a tendency to exhibit lower transdermal permeability relative to the free base of basic medicament. Therefore, an attempt to add a neutralizing agent such as sodium hydroxide to eliminate the acid addition salt in drug formulation has been made (Patent Document 2). Patent Document 2 describes that the problems of agglomeration or growth with time of metal salts caused by the neutralize reaction are solved by the use of an absorbent. However, the solution has not given a satisfying result for the improvement of transdermal permeability.

Patent Document 3 discloses transdermal tizanidine compositions, e.g. in the form of a patch.

### Prior Art Documents

### Patent Documents

Patent Document 1 : WO2009/066457
Patent Document 2 : WO2009/107479
Patent Document 3 : EP0315219

### SUMMARY OF THE INVENTION

### Problem to be Solved by the Invention

An object of the present invention is to provide a patch preparation having an excellent transdermal permeability of a medicament, comprising an acid addition salt of a basic medicament, i.e. tizanidine, as a raw material.

### Means for Solving the Problem

The present inventors have intensively investigated and found that the above problems can be solved by adding a compound capable of generating potassium ion to the adhesive layer containing an acid addition salt of a basic medicament, i.e. tizanidine, and a fatty acid-base ionic liquid. The acid which constitutes the acid addition salt is scavenged by potassium ion to generate a potassium salt.

More specifically, the present invention provides a patch preparation comprising:
a support and an adhesive layer on one surface of the support;
wherein the adhesive layer contains
a basic medicament, wherein the basic medicament is tizanidine,
a fatty acid-based ionic liquid, and
a potassium salt and/or potassium ion, wherein the potassium salt and/or potassium ion are generated as a result of reaction of an acid addition salt of tizanidine with a compound capable of generating potassium ion in the adhesive layer;
and wherein the term "fatty acid" refers to an acid selected from the group consisting of capric acid, sorbic acid, levulinic acid, lauric acid, myristic acid,palmitic acid, stearic acid, isostearic acid and oleic acid.

The invention further provides a composition for a patch preparation comprising:
an acid addition salt of a basic medicament, wherein the basic medicament is tizanidine;
a fatty acid-based ionic liquid; and
a compound capable of generating potassium ion;
and wherein the term "fatty acid" refers to an acid selected from the group consisting of capric acid, sorbic acid, levulinic acid, lauric acid, myristic acid, palmitic acid, stearic acid, isostearic acid and oleic acid.

### Effect of the Invention

The present invention provides a patch preparation containing a basic medicament, i.e. tizanidine, which produces an excellent transdermal absorbability of the basic medicament.

### DETAILED DESCRIPTION OF THE PREFERRED EMBODIMENTS

### Basic medicament

The basic medicament can utilize tizanidine, which is generically distributed in the form of an acid addition salt thereof. Examples of the acid addition salt can include an inorganic acid salt such as hydrochloride salt, sulfate salt, and hydrobromate salt; and an organic acid salt such as fumarate, maleate, citrate, and tartrate. The acid addition salt of the present invention is preferably the inorganic acid salt, and most preferably hydrochloride salt. When tizanidine is used as hydrochloride salt thereof, the potassium salt included in the adhesive layer should be potassium chloride.

### Compound capable of generating potassium ion

The potassium salt included in the adhesive layer of the patch preparation of the present disclosure is produced in the adhesive layer during or after the manufacturing process, as a result of reaction of the acid addition salt of basic medicament with a compound capable of generating potassium ion. Examples of the compound capable of generating potassium ion can include potassium hydroxide; potassium salt of an organic acid such as potassium citrate, potassium acetate, potassium tartrate, potassium lactate, and potassium salt of C₄₋₂₀ fatty acid; and potassium salt of an inorganic acid such as potassium bicarbonate, potassium dihydrogen phosphate, dibasic potassium phosphate, and tripotassium phosphate. Examples of the potassium salt of C₄₋₂₀ fatty acid include potassium sorbate, potassium oleate, and potassium levulinate. In some embodiments, potassium hydroxide and potassium salt of a fatty acid can be used alone or in combination.

In the past, a sodium compound such as sodium hydroxide has been considered as a preferred neutralizing agent for neutralizing an acid addition salt of a basic medicament to generate its free base in drug formulations. The fatty acid-based ionic liquid is known not only as an excellent dissolution adjuvant but also as a percutaneous absorption accelerator for medicaments. There are some cases where a basic medicament does not sufficiently dissolve in a solvent containing a fatty acid-based ionic liquid, when an acid addition salt of the basic medicament and a sodium compound such as sodium hydroxide are tried to dissolve in the solvent. Even if the basic medicament is dissolved, a patch preparation prepared using a sodium compound has less skin permeability than a patch preparation prepared using a potassium compound. The insufficient elimination of the acid addition salt is considered as one of the causes of the less skin permeability. In contrast, an acid addition salt of a basic medicament easily dissolves in an organic solvent containing a fatty-acid based ionic liquid without any residue in the presence of a compound capable of generating potassium ion. A patch preparation prepared using the obtained solution has a far superior skin permeability to a patch preparation prepared using a sodium compound.

The concentration of the compound capable of generating potassium ion can be within a range of about 0.6 mole to about 4.5 moles, about 0.8 mole to 2.0 moles, or about 0.8 mole to about 1.5 moles per mole of the acid contained in the acid addition salt of basic medicament.

### Fatty acid-based ionic liquid

The fatty acid-based ionic liquid is a salt of a fatty acid and an organic amine compound. The fatty acid-based ionic liquid can be formed in adhesive composition by adding a fatty acid and an organic amine compound to the adhesive composition, though it can also be prepared prior to its formation in the adhesive composition. As the fatty acid, C₅₋₂₀ saturated or unsaturated fatty acid can be utilized. Specific examples thereof can include capric acid, sorbic acid, levulinic acid, lauric acid, myristic acid, palmitic acid stearic acid, isostearic acid, oleic acid. As the organic amine compound, C₄₋₉ alkanolamine can be utilized. Specific examples thereof can include monoethanolamine, monoisopropanolamine, diethanolamine, diisopropanolamine, triethanolamine, triisopropanolamine, and trishydroxylmethylaminomethane.

Examples of the fatty acid-based ionic liquid include a fatty acid-based ionic liquid containing diisopropanolamine such as equimolar salt of levulinic acid and diisopropanolamine, equimolar salt of capric acid and diisopropanolamine, equimolar salt of isostearic acid and diisopropanolamine, equimolar salt of oleic acid and diisopropanolamine, and equimolar salt of sorbic acid and diisopropanolamine; a fatty acid-based ionic liquid containing triethanolamine such as equimolar salt of levulinic acid and triethanolamine, equimolar salt of caproic acid and triethanolamine, equimolar salt of isostearic acid and triethanolamine, equimolar salt of oleic acid and triethanolamine, and equimolar salt of sorbic acid and triethanolamine; and a fatty acid-based ionic liquid containing diethanolamine such as equimolar salt of levulinic acid diethanolamine, equimolar salt of capric acid and diethanolamine, equimolar salt of isostearic acid and diethanolamine, equimolar salt of oleic acid and diethanolamine, and equimolar salt of sorbic acid and diethanolamine. The formulation or the patch preparation may contain one or more fatty acid-based ionic liquids.

The concentration of the fatty acid-based ionic liquid can be selected from a range of about 0.2 to about 12 moles, about 0.4 to about 5 moles, or about 0.5 to about 1.5 moles per mole of the basic medicament.

### Adhesive layer

Adhesive layer is composed of an appropriate adhesive polymer. Examples of the adhesive polymer can include an acrylic polymer, a rubber polymer, a silicone based polymer, and a vinyl ether polymer. In the present disclosure, a rubber polymer such as styrene-isoprene-styrene block copolymer, styrene-butadiene-styrene block copolymer, polyisoprene, polyisobutylene, and polybutadiene can be used. When a rubber polymer is used, the concentration thereof may be about 5% to about 40% by weight, or about 10% to about 30% by weight relative to the total weight of the adhesive layer.

Preferably, the adhesive layer composed of a rubber polymer further comprises tackifier resin and/or softener. Examples of the tackifier resin can include rosin ester, hydrogenated rosin ester, rosin maleate, alicyclic saturated hydrocarbon resin, terpene resin, and polyolefin resin. The concentration of the tackifier resin may be about 10% to about 35% by weight, about 20% to about 30% by weight, or about 22% to about 28% by weight relative to the total weight of the adhesive layer. Examples of the softener can include naphthenic process oil; vegetable oil such as camellia oil and castor oil; liquid rubber such as liquid polybutene and liquid isoprene; and liquid paraffin.

### Other transdermal absorptive accelerator

In some embodiments, the adhesive layer may further contains one or more organic solvents. In some embodiments, the organic solvent has a percutaneous absorption accelerating effect, and examples thereof include a fatty acid, an alcohol, and an ester. The fatty acid may be the same as a fatty acid used for preparing the above-mentioned fatty acid-based ionic liquid. The amount of the fatty acid can be about 0.4 mole to about 5 moles, about 0.5 mole to about 3 moles, or about 0.8 mole to about 1.5 moles per mole of the fatty acid-based ionic liquid. Also, the concentration of the fatty acid can be about 0.5 to about 10% by weight, or about 1.0 to about 6% by weight relative to the total weight of the adhesive layer.

Examples of the alcohol can include a monovalent alcohol such as capryl alcohol, lauryl alcohol, myristyl alcohol, cetyl alcohol, stearyl alcohol, and oleyl alcohol; a divalent alcohol such as propylene glycol, butylene glycol, polyethylene glycol; and a trivalent alcohol such as glycerin. The amount of the alcohol can be about 5% to about 30% by weight, about 8% to about 25% by weight, or about 10% to about 15% by weight relative to the total weight of the adhesive layer. In the present disclosure, oleyl alcohol, which exhibits an excellent transdermal permeability, can be used. The amount of oleyl alcohol can be about 3% to about 15% by weight or about 8% to about 12% by weight relative to the total weight of the adhesive layer. Oleyl alcohol can also be used in combination with the other alcohols.

Examples of the ester can include a carbonic ester such as propylene carbonate; and a fatty acid ester such as diethyl sebacate, isopropyl myristate, diisopyl adipate, myristyl permeate, stearyl stearate, and medium chain fatty acid triglyceride.

In some embodiments, the adhesive layer may further contain a filler. The use of a filler can improve not only the adhesive property of the adhesive layer but also the release of a drug. The amount of the filler can be about 0.5% to about 5% by weight relative to the total amount of the adhesive layer. Examples of the filler can include hydrous silica, fumed silica, talc, crystalline cellulose, starch, carmellose, metal salt of carmellose. In some embodiments, fumed silica can be used as the filler. In some embodiments, commercially-available fumed silica, AEROSIL^{®}, can be used.

The adhesive layer may further contain a commonly-used additive for the adhesive layer of a patch preparation such as softener and antioxidant.

### Preparation method of patch preparation

In some embodiments, a preparation method of the patch preparation of the present disclosure is provided. A fatty acid and an organic amine for forming a fatty acid-based ionic liquid are mixed together to yield a homogeneous solution. In some embodiments, an organic solvent may be added into the solution. The organic solvent may produce a percutaneous absorption accelerating effect. An acid addition salt of a basic medicament, i.e. tizanidine, and a compound capable of generating potassium ion are dissolved into the solution to form a composition for the patch preparation (a medicament composition). In some embodiments, the solution may be heated (e.g. about 45-60°C). In some embodiments, the fatty acid, the organic amine, the acid addition salt of the basic solution, and the compound capable of generating potassium ion may be mixed together at the same time.

In this medicament composition, the acid attached to the basic medicament is eliminated to generate the free base of the basic medicament. In addition, the eliminated acid and potassium ion therein are reacted to generate a potassium salt.

Although these reactions may be completed during the preparation of the basic medicament solution, some of the reactions can occur during the step of mixing the medicament composition with the material that forms the adhesive layer, during the step of coating the mixture onto the support, or during the step of drying the support. Additionally, some of the reactions can occur in the adhesive layer after the production process is completed. A patch preparation in which both an acid addition salt of a basic medicament and a potassium salt are contained in the adhesive layer is also within the scope of the present invention.

The medicament composition is prepared, and then the composition is mixed with the material that forms the adhesive layer. In some embodiments, the adhesive layer comprises a rubber polymer. For example, a rubber polymer and a tackifier resin are mixed with toluene and then heated (about 60°C) to yield a molten solution. The molten solution is mixed with the previously-prepared composition to yield a polymer solution. A filler is added thereto and mixed to form an adhesive composition. Then, the composition is coated on one surface of a support (e.g., a woven fabric, an unwoven fabric, or a PET film). The support is heated (about 80°C) and dried to remove toluene, thereby forming an adhesive layer on the support.

### EXAMPLES

Hereinafter, the present disclosure is explained in detail with examples. The present disclosure is not intended to be limited in any way by these examples.

### Patch preparation containing oxycodone (not according to the invention)

Patch preparations with the compositions (% by weight) shown in Table 1 were prepared. The transdermal permeability of the medicament in the prepared patch preparations was evaluated according to the skin permeability test using Franz cell. The skin of a pig was used in the test on the patch preparations. The cumulative skin permeation amounts at each sampling point are shown in Table 1.

**[Table 1]**

| | Ex.1-a | Ex.1-b | Ex.1-c | Ex.1-d | Ex.1-e | Com.1 |
|---|---|---|---|---|---|---|
| | M286 | M308 | M307 | M294 | | M313 |
| Oxyco.HCl 3H₂O | 3.461 | 3.461 | 3.461 | 3.461 | 3.461 | 3.461 |
| Potassium hydroxide | 0.55 | | | 0.55 | 0.55 | |
| Potassium sorbate | | 1.4 | 1.3 | | | |
| Sodium hydroxide | | | | | | 0.34 |
| Diisopropanolamine | 1.5 | 1.5 | 1.5 | | 0.7 | 1.5 |
| Triethanolamine | | | | 2.2 | | |
| Levulinic acid | 1.0 | | 0.8 | 1.0 | 1.0 | 1.0 |
| Oleic acid | 4.0 | 4.0 | 3.0 | 4.0 | 4.0 | 4.0 |
| Oleyl alcohol | 10.0 | 10.0 | 10.0 | 8.0 | 10.0 | 10.0 |
| Glycerin | 3.0 | 4.0 | 4.0 | 2.5 | | 3.0 |
| Concentrated glycerin | | | | | 3.0 | |
| Propylene carbonate | 4.0 | 5.0 | 5.0 | 2.5 | 5.0 | 5.0 |
| Medium-chain triglyceride | 10.0 | 10.0 | 10.0 | 10.0 | 10.0 | 10.0 |
| Liquid paraffin | 17.34 | 16.49 | 16.81 | 17.64 | 19.14 | 17.55 |
| AEROSIL^{®} | 3.0 | 3.0 | 3.0 | 3.0 | 3.0 | 3.0 |
| PX-1150N | 27.0 | 26.0 | 26.0 | 27.0 | 26.0 | 26.0 |
| SIS-5002 | 15.0 | 15.0 | 15.0 | 15.0 | 14.0 | 15.0 |
| Sodium pyrosulfite | 0.1 | 0.1 | 0.1 | 0.1 | 0.1 | 0.1 |
| Propyl gallate | 0.05 | 0.05 | 0.05 | 0.05 | 0.05 | 0.05 |
| Total | 100 | 100 | 100 | 97.00 | 100 | 100 |
| Cumulative skin permeation amount after 2hrs (µg/cm²) | 1.72 | | | | 0.5 | 0.3 |
| Cumulative skin permeation amount after 4hrs (µg/cm²) | 12.5 | 2.2551 | 3.9967 | 1.86 | 5 | 1.8 |
| Cumulative skin permeation amount after 6hrs (µg/cm²) | 40.32 | 7.0518 | 15.8 | 6.015 | 14.8 | 4 |
| Cumulative skin permeation amount after 8hrs (µg/cm²) | 76.469 | 16.639 | 33.5 | 1 1.982 | 31.1 | 7.3 |
| Cumulative skin permeation amount after 24hrs (µg/cm²) | 245.88 | 164.53 | 224.45 | 90.235 | 248.3 | 52.5 |
| (acid)/(base) | 1.08 | 1.14 | 1.32 | 0.93 | 1.51 | 1.15 |

It was shown that the patch preparations of Examples 1-a to 1-e containing a compound capable of generating potassium ion as raw material produced an excellent skin permeability of the medicament. The patch preparation of Comparative Example 1 containing sodium hydroxide instead of potassium hydroxide had less skin permeability. The patch preparation of Example 1-d had less skin permeability than those of Examples 1-a to 1-c. It is considered because the patch of Example 1-d doesn't include fatty acid-based ionic liquid containing diisopropanolamine. Patch preparation of Example 1-a and 1-c had especially excellent skin permeability. It is considered because they contain both levulinic acid-diisopropanolamine and oleic acid-diisopropanolamine.

### Patch preparation containing hydromorphone (not according to the invention)

Patch preparations with the compositions (% by weight) shown in Table 2 were prepared. The transdermal permeability of the medicament in the prepared patch preparations was evaluated according to the skin permeability test using Franz cell. The skin of a pig was used in the test on the patch preparations. The cumulative skin permeation amounts at each sampling point are shown in Table 2.

**[Table 2]**

| | Ex. 2-a | Ex. 2-b | Com. 2-a | Ex. 2-c | Ex. 2-d | Ex. 2-e | Ex. 2-f | Com.2 -b |
|---|---|---|---|---|---|---|---|---|
| | N511 | N488 | S805 | S842 | S847 | S846 | S827 | S826 |
| Hydromorphone hydrochloride | 1.0 | 1.0 | 1.0 | 3.0 | 3.0 | 3.0 | 3.0 | 3.0 |
| Potassium hydroxide | | | | | 1 | 0.4 | 0.61 | |
| Potassium sorbate | 0.5 | 0.2 | | 1.4 | | 1.4 | | |
| Diisopropanolamine | 1.5 | 1.5 | 1.5 | 1.5 | 1.5 | 1.5 | 1.5 | 1.5 |
| Triethanolamine | 4.0 | 4.7 | 1.5 | | | | 1.5 | 1.4 |
| Levulinic acid | | | | | 1.1 | | 1.1 | 1.1 |
| Oleic acid | 6.0 | 6.0 | 6.0 | 3.0 | 3.0 | 3.0 | 6.0 | 3.0 |
| Sorbic acid | 2.7 | 0.2 | | | | | | |
| Lactic acid | | 2.0 | | | | | | |
| Sodium lactate | | 1.6 | | | | | | |
| Oleyl alcohol | | | 5.0 | 10.0 | 10.0 | 10.0 | 8.0 | 8.0 |
| Propylene glycol | 5 | | | | | | | |
| PEG200 | | 2.0 | | | | | | |
| Glycerin | 7.0 | 7.0 | 5.0 | 4.0 | 4.0 | 4.0 | 3.0 | 4.0 |
| Propylene carbonate | 5.0 | 5.0 | 5.0 | 5.0 | 5.0 | 5.0 | 2.5 | 5.0 |
| Medium-chain triglyceride | | | | 10.0 | 10.0 | 10.0 | 10.0 | 10.0 |
| Diethyl sebacate | 5.0 | 5.0 | 5.0 | | | | | |
| Isopropyl myristate | 5.0 | 5.0 | 5.0 | | | | | |
| Liquid paraffin | 12.15 | 14.65 | 18.85 | 17.95 | 17.27 | 17.55 | 17.64 | 17.85 |
| AEROSIL^{®} | 4.0 | 3.0 | 3.0 | 3.0 | 3.0 | 3.0 | 3.0 | 3.0 |
| PX-1150N | 27.0 | 27.0 | 28.0 | 26.0 | 26.0 | 26.0 | 27.0 | 27.0 |
| SIS-5002 | 14.0 | 14.0 | 15.0 | 15.0 | 15.0 | 15.0 | 15.0 | 15.0 |
| Sodium pyrosulfite | 0.1 | 0.1 | 0.1 | 0.1 | 0.1 | 0.1 | 0.1 | 0.1 |
| Propyl gallate | 0.05 | 0.05 | 0.05 | 0.05 | 0.05 | 0.05 | 0.05 | 0.05 |
| Total | 100.0 | 100.0 | 100.0 | 100.0 | 100.0 | 100.0 | 100.0 | 100.0 |
| Cumulative skin permeation amount after 2hrs (µg/cm²) | | | | 0.3198 | | | 0.9786 | |
| Cumulative skin permeation amount after 4hrs (µg/cm²) | 0.472 | 0.348 | 0.965 | 0.5907 | | 0.6 | 5.759 | 0.458 |
| Cumulative skin permeation amount after 8hrs (µg/cm²) | 2.941 | 3.095 | 6.107 | 10.274 | 3.9 | 7.5 | 25.721 | 7.3018 |
| Cumulative skin permeation amount after 24hrs (µg/cm²) | 36.21 | 27.88 | 15.33 | 68.594 | 71.9 | 87.8 | 74.524 | 38.435 |
| (acid)/(base) | 1.36 \| | 0.79 | 1.11 | 0.97 | 0.69 | 0.72 | 1.02 | 1.08 |

The patch preparations of Examples 2-a and 2-b containing potassium sorbate produced a higher skin permeability of the medicament than that of Comparative Example 2-a containing no compound capable of generating potassium ion. The patch preparations of Examples 2-c to 2-f containing a compound capable of generating potassium ion produced a higher skin permeability of the medicament than that of Comparative Example 2-b containing no compound capable of generating potassium ion.

### Patch preparation containing tizanidine hydrochloride

Patch preparations with the compositions (% by weight) shown in Table 3 were prepared. The transdermal permeability of the medicament in the prepared patch preparations was evaluated according to the skin permeability test using Franz cell. The skin of a pig was used in the test on the patch preparations. The cumulative skin permeation amounts at each sampling point are shown in Table 3.

**[Table 3]**

| | Ex. 3-a | Ex. 3-b | Ex. 3-c | Ex. 3-d | Ex. 3-e | Ex. 3-f |
|---|---|---|---|---|---|---|
| | T651 | T695 | T697 | T700 | T702 | T696 |
| Tizanidine hydrochloride | 1.72 | 1.72 | 1.72 | 1.72 | 1.72 | 1.72 |
| Sorbic acid | 0.66 | | | | | |
| Potassium sorbate | 0.89 | 0.89 | 0.89 | 0.89 | 0.89 | 0.89 |
| Oleic acid | | 1.50 | 1.50 | 1.50 | 1.50 | 1.50 |
| Triethanolamine | 0.5 | 0.5 | 0.4 | | | |
| Oleyl alcohol | 10.0 | 10.0 | 10.0 | 10.0 | 10.0 | 10.0 |
| Myristyl alcohol | | 3.0 | 3.0 | | 3.0 | 3.0 |
| Glycerin | 5.0 | 5.0 | 5.0 | 5.0 | 5.0 | 5.0 |
| DiPG | 4.0 | 4.0 | 4.0 | 4.0 | 4.0 | 4.0 |
| BG | 3.0 | 3.0 | 3.0 | 3.0 | 3.0 | 3.0 |
| Medium-chain triglyceride | \| 5.0 | \| | | | | |
| AEROSIL^{®} | 3.0 | 3.0 | 3.0 | 3.0 | 3.0 | 3.0 |
| Liquid paraffin | 18.08 | 23.24 | 23.34 | 26.74 | 23.74 | 23.74 |
| Terpene resin | 32.00 | 28.00 | 28.00 | 28.00 | 28.00 | 28.00 |
| SIS-5002 | 16.00 | 16.00 | 16.00 | 16.00 | 16.00 | 16.00 |
| Sodium sulfite | | 0.10 | 0.10 | 0.10 | 0.10 | 0.10 |
| Sodium pyrosulfite | 0.10 | | | | | |
| Propyl gallate | 0.05 | 0.05 | 0.05 | 0.05 | 0.05 | 0.05 |
| Total | 100 | 100 | 100 | 100 | 100 | 100 |
| Cumulative skin permeation amount after 4hrs (µg/cm²) | 0.4 | 2.1 | 1.9 | 3.6 | 0.8 | 2.4 |
| Cumulative skin permeation amount after 6hrs (µg/cm²) | 1.6 | 5.8 | 5.7 | 7.0 | 2.7 | 8.5 |
| Cumulative skin permeation amount after 8hrs (µg/cm²) | 4.5 | 10.5 | 11.0 | 12.0 | 6.1 | 17.7 |
| Cumulative skin permeation amount after 22hrs (µg/cm²) | 84.0 | 51.2 | 54.5 | 68.7 | 57.8 | 105.2 |
| Cumulative skin permeation amount after 24hrs (µg/cm²) | 98.5 | 56.1 | 58.9 | 77.3 | 66.4 | 112.5 |

The patch preparations of Examples 3-a to 3-f containing potassium sorbate which is a compound capable of generating potassium ion exhibited an excellent skin permeability of the medicament.

### Industrial Applicability

The patch preparation of the present invention can produce an excellent skin permeability of a medicament.

## Claims

1. A patch preparation comprising:
a support and an adhesive layer on one surface of the support;
wherein the adhesive layer contains
a basic medicament, wherein the basic medicament is tizanidine,
a fatty acid-based ionic liquid, and
a potassium salt and/or potassium ion, wherein the potassium salt and/or potassium ion are generated as a result of reaction of an acid addition salt of tizanidine with a compound capable of generating potassium ion in the adhesive layer;
and wherein the term "fatty acid" refers to an acid selected from the group consisting of capric acid, sorbic acid, levulinic acid, lauric acid, myristic acid, palmitic acid, stearic acid, isostearic acid and oleic acid.

2. The patch preparation according to claim 1, wherein the acid addition salt of tizanidine is tizanidine hydrochloride.

3. The patch preparation according to claim 1 or 2, wherein the fatty acid-based ionic liquid is a salt of tizanidine or tizanidine hydrochloride and a fatty acid.

4. The patch preparation according to claim 1 or 2, wherein the fatty acid-based ionic liquid is a salt of capric acid, sorbic acid, levulinic acid, lauric acid, myristic acid, palmitic acid, stearic acid, isostearic acid, or oleic acid and monoethanolamine, monoisopropanolamine, diethanolamine, diisopropanolamine, triethanolamine, triisopropanolamine, or trishydroxylmethylaminomethane.

5. The patch preparation according to any one of claims 1, 2 and 4, wherein the fatty acid-based ionic liquid is a salt selected from the group consisting of oleic acid-diisopropanolamine, levulinic acid-diisopropanolamine, sorbic acid-diisopropanolamine, and a combination thereof.

6. The patch preparation according to any one of claims 1-5, wherein the potassium salt is potassium chloride.

7. A composition for a patch preparation comprising:
an acid addition salt of a basic medicament, wherein the basic medicament is tizanidine;
a fatty acid-based ionic liquid; and
a compound capable of generating potassium ion;
and wherein the term "fatty acid" refers to an acid selected from the group consisting of capric acid, sorbic acid, levulinic acid, lauric acid, myristic acid, palmitic acid, stearic acid, isostearic acid and oleic acid.

8. The composition according to claim 7, wherein the acid addition salt of tizanidine is tizanidine hydrochloride.

9. The composition according to claim 7 or 8, wherein the fatty acid-based ionic liquid is a salt of tizanidine or tizanidine hydrochloride and a fatty acid.

10. The composition according to claim 7 or 8, wherein the fatty acid-based ionic liquid is a salt of capric acid, sorbic acid, levulinic acid, lauric acid, myristic acid, palmitic acid, stearic acid, isostearic acid, or oleic acid and monoethanolamine, monoisopropanolamine, diethanolamine, diisopropanolamine, triethanolamine, triisopropanolamine, or trishydroxylmethylaminomethane.

## Patentansprüche

1. Ein Pflasterpräparat, umfassend:
einen Träger und eine Klebeschicht auf einer Oberfläche des Trägers;
wobei die Klebeschicht enthält
ein basisches Medikament, wobei das basische Medikament Tizanidin ist,
eine ionische Flüssigkeit auf Fettsäurebasis und
ein Kaliumsalz und/oder Kaliumion, wobei das Kaliumsalz und/oder Kaliumion als Ergebnis der Reaktion eines Säureadditionssalzes von Tizanidin mit einer Verbindung, die in der Lage ist, in der Klebeschicht Kaliumionen zu erzeugen, erzeugt wird;
und wobei sich der Begriff "Fettsäure" auf eine Säure, ausgewählt aus der Gruppe bestehend aus Caprinsäure, Sorbinsäure, Lävulinsäure, Laurinsäure, Myristinsäure, Palmitinsäure, Stearinsäure, Isostearinsäure und Oleinsäure, bezieht.

2. Das Pflasterpräparat gemäß Anspruch 1, wobei das Säureadditionssalz des Tizanidins Tizanidinhydrochlorid ist.

3. Das Pflasterpräparat gemäß Anspruch 1 oder 2, wobei die ionische Flüssigkeit auf Fettsäurebasis ein Salz von Tizanidin oder Tizanidinhydrochlorid und einer Fettsäure ist.

4. Das Pflasterpräparat gemäß Anspruch 1 oder 2, wobei die ionische Flüssigkeit auf Fettsäurebasis ein Salz von Caprinsäure, Sorbinsäure, Lävulinsäure, Laurinsäure, Myristinsäure, Palmitinsäure, Stearinsäure, Isostearinsäure oder Oleinsäure und Monoethanolamin, Monoisopropanolamin, Diethanolamin, Diisopropanolamin, Triethanolamin, Triisopropanolamin oder Trishydroxylmethylaminomethan ist.

5. Das Pflasterpräparat gemäß einem der Ansprüche 1, 2 und 4, wobei die ionische Flüssigkeit auf Fettsäurebasis ein Salz, ausgewählt aus der Gruppe bestehend aus Oleinsäure-Diisopropanolamin, Lävulinsäure-Diisopropanolamin, Sorbinsäure-Diisopropanolamin und eine Kombination davon ist.

6. Das Pflasterpräparat gemäß einem der Ansprüche 1 bis 5, wobei das Kaliumsalz Kaliumchlorid ist.

7. Eine Zusammensetzung für ein Pflasterpräparat, umfassend:
ein Säureadditionssalz eines basischen Medikaments, wobei das basische Medikament Tizanidin ist;
eine ionische Flüssigkeit auf Fettsäurebasis; und
eine Verbindung, welche in der Lage ist, Kaliumionen zu erzeugen;
und wobei sich der Begriff "Fettsäure" auf eine Säure, ausgewählt aus der Gruppe bestehend aus Caprinsäure, Sorbinsäure, Lävulinsäure, Laurinsäure, Myristinsäure, Palmitinsäure, Stearinsäure, Isostearinsäure und Oleinsäure, bezieht.

8. Die Zusammensetzung gemäß Anspruch 7, wobei das Säureadditionssalz des Tizanidins Tizanidinhydrochlorid ist.

9. Die Zusammensetzung gemäß Anspruch 7 oder 8, wobei die ionische Flüssigkeit auf Fettsäurebasis ein Salz von Tizanidin oder Tizanidinhydrochlorid und einer Fettsäure ist.

10. Die Zusammensetzung gemäß Anspruch 7 oder 8, wobei die ionische Flüssigkeit auf Fettsäurebasis ein Salz von Caprinsäure, Sorbinsäure, Lävulinsäure, Laurinsäure, Myristinsäure, Palmitinsäure, Stearinsäure, Isostearinsäure oder Oleinsäure und Monoethanolamin, Monoisopropanolamin, Diethanolamin, Diisopropanolamin, Triethanolamin, Triisopropanolamin oder Trishydroxylmethylaminomethan ist.

## Revendications

1. Préparation de patch comprenant :
un support et une couche adhésive sur une surface du support ;
dans laquelle la couche adhésive contient
un médicament basique, ledit médicament basique étant la tizanidine,
un liquide ionique à base d'acide gras, et
un sel de potassium et/ou un ion potassium, lesdits sel de potassium et/ou ion potassium étant générés en résultat de la réaction d'un sel d'addition d'acide de tizanidine avec un composé capable de générer un ion potassium dans la couche adhésive ;
et dans laquelle l'expression "acide gras" se réfère à un acide choisi dans le groupe constitué par l'acide caprique, l'acide sorbique, l'acide lévulinique, l'acide laurique, l'acide myristique, l'acide palmitique, l'acide stéarique, l'acide isostéarique et l'acide oléique.

2. Préparation de patch selon la revendication 1, dans laquelle le sel d'addition d'acide de tizanidine est le chlorhydrate de tizanidine.

3. Préparation de patch selon la revendication 1 ou 2, dans laquelle le liquide ionique à base d'acide gras est un sel de tizanidine ou de chlorhydrate de tizanidine et d'un acide gras.

4. Préparation de patch selon la revendication 1 ou 2, dans laquelle le liquide ionique à base d'acide gras est un sel d'acide caprique, d'acide sorbique, d'acide lévulinique, d'acide laurique, d'acide myristique, d'acide palmitique, d'acide stéarique, d'acide isostéarique, ou d'acide oléique, et de monoéthanolamine, de monoisopropanolamine, de diéthanolamine, de diisopropanolamine, de triéthanolamine, de triisopropanolamine, ou de trishydroxylméthylaminométhane.

5. Préparation de patch selon l'une quelconque des revendications 1, 2 et 4, dans laquelle le liquide ionique à base d'acide gras est un sel choisi dans le groupe constitué par l'acide oléique-diisopropanolamine, l'acide lévulinique-diisopropanolamine, l'acide sorbique-diisopropanolamine, et une combinaison de ceux-ci.

6. Préparation de patch selon l'une quelconque des revendications 1 à 5, dans laquelle le sel de potassium est le chlorure de potassium.

7. Composition pour une préparation de patch, comprenant :
un sel d'addition d'acide d'un médicament basique, ledit médicament basique étant la tizanidine ;
un liquide ionique à base d'acide gras ; et
un composé capable de générer un ion potassium ;
et dans laquelle l'expression "acide gras" se réfère à un acide choisi dans le groupe constitué par l'acide caprique, l'acide sorbique, l'acide lévulinique, l'acide laurique, l'acide myristique, l'acide palmitique, l'acide stéarique, l'acide isostéarique et l'acide oléique.

8. Composition selon la revendication 7, dans laquelle le sel d'addition d'acide de tizanidine est le chlorhydrate de tizanidine.

9. Composition selon la revendication 7 ou 8, dans laquelle le liquide ionique à base d'acide gras est un sel de tizanidine ou de chlorhydrate de tizanidine et d'un acide gras.

10. Composition selon la revendication 7 ou 8, dans laquelle le liquide ionique à base d'acide gras est un sel d'acide caprique, d'acide sorbique, d'acide lévulinique, d'acide laurique, d'acide myristique, d'acide palmitique, d'acide stéarique, d'acide isostéarique, ou d'acide oléique, et de monoéthanolamine, de monoisopropanolamine, de diéthanolamine, de diisopropanolamine, de triéthanolamine, de triisopropanolamine, ou de trishydroxylméthylaminométhane.
